# EUROPÄISCHE PATENTSCHRIFT

(11) **EP 2 533 763 B1**
(45) Veröffentlichungstag und Bekanntmachung des Hinweises auf die Patenterteilung: **12.07.2023**
(21) Anmeldenummer: 11705444.5
(22) Anmeldetag: 11.02.2011
(51) Int. Cl.: A61K 9/107, A61K 9/113, A61K 47/02, A61K 47/06, A61K 47/26, A61K 47/44, A61P 17/00, A61P 29/00, A61P 43/00, C12N 15/113

(54) **DERMATOLOGISCHE, PHARMAZEUTISCHE ZUSAMMENSETZUNG GEEIGNET FÜR OLIGONUKLEOTIDE**
DERMATOLOGICAL, PHARMACEUTICAL COMPOSITION SUITABLE FOR OLIGONUCLEOTIDES
COMPOSITION PHARMACEUTIQUE DERMATOLOGIQUE ADAPTÉE AUX OLIGONUCLÉOTIDES

(30) Priorität: 10.02.2010 DE 102010007562
(43) Veröffentlichungstag der Anmeldung: 19.12.2012
(73) Patentinhaber: Sterna Biologicals GmbH, 35037 Marburg (DE)
(72) Erfinder: SCHMIDTS, Thomas, 35394 Gießen (DE); GARN, Holger, 35037 Marburg (DE); RUNKEL, Frank, 35418 Buseck (DE)
(74) Vertreter: Patentanwälte Olbricht Buchhold Keulertz
(86) Internationale Anmeldenummer: PCT/EP2011/000648
(87) Internationale Veröffentlichungsnummer: WO 2011/098285

(56) Entgegenhaltungen:
- WO-A1-00/09673
- DE-A1- 10 238 298
- DE-A1- 10 346 487
- DE-A1-102004 010 547
- DE-A1-102007 020 554
- US-B1- 6 841 539

## Beschreibung

Die vorliegende Erfindung betrifft eine pharmazeutische Zubereitung für die topische Anwendung. Insbesondere betrifft die Erfindung Emulsionen mit dispergierter, innenliegender, diskontinuierlicher Wasserphase, mit der Oligonukleotide als Mittel gegen entzündliche Erkrankungen zur topischen Anwendung formuliert und appliziert werden.

### Stand der Technik

Oligonukleotide sind aus wenigen Nukleotiden (DNA- oder RNA-Bausteine) aufgebaute Stoffe, deren Nukleotidsequenz im Allgemeinen aus ca. 10-100 Nukleotideinheiten besteht. Oligonukleotide sind z.B. als Primer, die in der Polymerase-Kettenreaktion (PCR) eingesetzt werden, bekannt. Antisense-Oligonukleotide sind Oligonukleotide, deren Basensequenz komplementär zu einer zellulären, viralen oder synthetischen RNA oder DNA ist und die diese über Watson-Crick-Basenpaarung binden können. Häufig werden solche Moleküle gegen funktionale mRNAs gerichtet. Durch die spezifische Bindung an die mRNA wird durch Blockade der Translation letztlich die Bildung des betreffenden Proteins, das von dieser mRNA kodiert wird, verhindert. Antisense-Moleküle können verschiedenen Molekülklassen zugehören, zu denen antisense DNA (asDNA), "small inhibitory" RNA (siRNA), Ribozyme und DNAzyme gehören. Die letzten beiden Molkülgruppen zeichnen sich durch eine inhärente katalytische Aktivität aus, die u.a. zu einer direkten Spaltung der gebundenen Ziel-RNA führen kann.

Beispielhaft aber nicht abschließend sind als DNAzyme die DNAzyme der DE 103 46 487.5 genannt, die zur Herstellung eines Mittels gegen entzündliche Erkrankungen offenbart sind.

Es ist allgemein bekannt, dass Oligonukleotide sehr sensitiv gegenüber natürlich vorkommenden oder rekombinanten Nukleasen sind. Nukleasen sind eine Gruppe von Enzymen, die ubiquitär vorkommen und meist als Hydrolasen an Esterbindungen wirken und den Abbau und die Degradation von Oligonukleotiden katalysieren. Dem Fachmann sind Nukleasen als DNAsen oder RNAsen bekannt.

Die Sensitivität gegenüber Nukleasen ist vor allem beim medizinischen Einsatz der Oligonukleotide problematisch. Im Vergleich zu klassischen Arzneistoffen können Oligonukleotide rasch durch DNAsen und RNAsen abgebaut werden, was zu einer kurzen Halbwertszeit und damit zu einer geringeren Bioverfügbarkeit in den Zielzellen führt.

Damit Oligonukleotide auch medizinisch (insbesondere therapeutisch) einsetzbar sind, müssen sie wirksam vor den Nukleasen geschützt werden. Dazu werden weltweit mit verschiedenen technischen Ansätzen große Anstrengungen unternommen, z.B. die Struktur der Oligonukleotide durch chemische Modifikationen zu verändern, um die Stabilität zu erhöhen oder Arzneistoffträgersysteme sogenannte Drug-Delivery-Systeme zu entwickeln. Zu diesen Drug-Delivery-Systemen gehören z.B. Liposomen, Nanopartikel, virale Hüllkapside und Protamin Oligonukleotid Partikel.

Viele dieser Drug-Delivery-Systeme weisen Nachteile auf, z.B. schützen sie nicht ausreichend wirksam gegenüber dem Abbau durch Nukleasen, weisen nur eine schlechte Aufnahme in die Zielgewebe und -zellen auf, oder beinhalten toxische oder störende Bestandteile, die die Wirksamkeit der Oligonukleotide beinträchtigen. Besonders für die topische Anwendung gibt es derzeit keine geeignete Zubereitung, mit der Oligonukleotide, wie z.B. DNAzyme, formuliert und als Mittel gegen entzündliche Erkrankungen appliziert werden können, da keine ausreichenden Schutzmöglichkeiten gegen Bakterien, Pilze und Nukleasen vorhanden sind. Der erfolgreiche Einsatz von Oligonukleotiden in der Dermatologie wird bisher u.a. durch diese fehlenden Schutzmöglichkeiten verhindert.

### Aufgabe

Aufgabe der vorliegenden Erfindung ist es, die Nachteile im Stand der Technik zu beseitigen und eine Zubereitung für die topische Anwendung von Oligonukleotiden bereitzustellen.

### Lösung

Die Aufgabe wird erfindungsgemäß gelöst durch die Ansprüche, insbesondere durch eine Pharmazeutische Zusammensetzung gemäß dem Anspruch 1. Darin wird eine Emulsion mit dispergierter, innenliegender, diskontinuierlicher Wasserphase definiert.

Überraschenderweise wurde eine stabile wirksame Formulierung in Form einer Emulsion mit dispergierter, innenliegender, diskontinuierlicher Wasserphase gefunden, die Oligonukleotide stabil hält, wirksam vor enzymatischen Abbau durch Nukleasen schützt und eine gute Aufnahme in die Zielgewebe und -zellen ermöglicht.

### 1. Charakterisierung der Formulierung

Die erfindungsgemäße Formulierung in Form einer Emulsion mit dispergierter, innenliegender, diskontinuierlicher Wasserphase entsteht durch Verwendung der galenischen Form Wasser-in-Öl-in-Wasser Emulsion, sogenannter W-O-W-Emulsion, bzw. Wasser-in-Öl Emulsion, sogenannter W-O-Emulsionen, unter Zusatz von Magnesium- oder Natriumionen. Überraschenderweise hat sich gezeigt, dass der Zusatz von Magnesium- oder Natriumionen zur Formulierung für die Wirkung der Oligonukleotide sehr vorteilhaft ist, die Stabilität der Oligonukleotide durch den Schutz vor enzymatischem Abbau verbessert und damit eine gute Aufnahme in die Zielzellen z.B. Hautzellen, Lungen- und Darmepithelzellen, Schleimhaut, Nasenepithelzellen und Zellen des Rachenraums, ermöglicht.

### Stoffe der erfindungsgemäßen Formulierung

Die erfindungsgemäße Formulierung umfasst
- mindestens einen lipophilen Emulgator ausgewählt aus der Gruppe der Sorbitan Fettsäureester und Glycerol-Derivate, wobei die Glycerol-Derivate ausgewählt sind aus der Gruppe bestehend aus Glycerol Stearate, Glycerol dioleate und Glycerolmonooleate,
- einen hydrophilen Emulgator ausgewählt aus der Gruppe bestehend aus Polysorbate, ethoxylierte Polyethylenglycole, etoxylierte Ether und etoxylierte Ester,
- ein Phospholipid als amphiphile Komponente,
- mindestens einen Konsistenzgeber,
- mindestens einen gesättigten Kohlenwasserstoff ausgewählt aus der Gruppe Paraffin und Polysiloxane als okklusive Komponente oder alternativ ein synthetisches oder natürliches Öl oder Wax,
- mindestens einen anorganischen und/oder organischen Zusatz umfassend ein Salz oder eine ionische Flüssigkeit und mindestens ein Oligonukleotid.

Die Zusammensetzung ist dadurch gekennzeichnet, dass sie eine Wasser-in-Öl-Wasser (W-O-W) Emulsion oder Wasser-in-Öl (W-O)-Emulsion mit dispergierter, innenliegender, diskontinuierlicher Wasserphase, unter Zusatz von Magnesium- oder Natriumionen, ist und das Oligonukleotid ein DNAzym ist, ausgewählt aus der Gruppe bestehend aus den DNAzymen hgd1 bis hgd70 und td1 bis td78.

Alternativ umfasst die erfindungsgemäße Formulierung zusätzlich mindestens einen Feuchthaltefaktor.

Alternativ umfasst die erfindungsgemäße Formulierung zusätzlich mindestens einen Konservierungsstoff.

Der mindestens eine Konsistenzgeber ist ausgewählt aus der Gruppe der Fettsäureester (z.B. Cetylpalmitat, Myristylmyristate) Polyethylenglycole, Cera Alba, mikrokristallines Wachs, Wollwachs und seine Alkohole, Hydrogenated Castor Oil, Carbomere (z.B. quervernetzte Acrylsäurepolymere) oder Cellulose.

Die mindestens eine okklusive Komponente ist ausgewählt aus der Gruppe der gesättigten Kohlenwasserstoffe, wie z.B. Paraffin oder Polysiloxane, wie Silikonöle.

Der mindestens eine anorganische und/oder organische Zusatz ist ein Salz oder eine ionische Flüssigkeit deren Kationenanteil Na, Mg, K, Li, Ca, Fe, Cu, Ag oder eine Kombination dieser Elemente (z.B. Gemisch aus NaCl, MgSO₄) oder eine Kombination der Elemente mit organischen Kationen (z.B. Gemisch aus Mg(N(SO₂CF₃)₂)₂, Mg(OSO₂CF₃)₂ in einer ionischen Flüssigkeit wie z.B. 1-Ethyl-3-methylimidazolium Chlorid, 1-Butyl-3-methylimidazolium Tetrafluoroborat, 1-Ethyl-3-methylimidazolium Sulfat, 1-Ethyl-3-methylimidazolium Trifluoromethansulfonat, 1-Ethyl-3-methylimidazolium dicyanamid oder 1-Ethyl-1-methylpyrrolidinium bis(trifluoromethylsulfonyl)amid) umfasst.

Der mindestens eine hydrophile Emulgator ist ausgewählt aus der Gruppe der Polysorbate oder ethoxylierte Polyethylenglycole (wie z.B. Tweens, Steareths, Laureths, Ceteareths) oder etoxylierte Ether bzw. Ester.

Die mindestens eine amphiphile Komponente ist ausgewählt aus der Gruppe der Phospholipide, vorzugsweise Lecithin.

Der mindestens eine Feuchthaltefaktor ist ausgewählt aus der Gruppe Glycerin, Polyole, Osmolyte.

Das mindestens eine Öl ist ausgewählt aus der Gruppe der veresterten Fettsäuren (z.B. Decyl oleate), Wachse (z.B. Jojobawax) oder partiell veresterte Glyceride (z.B. mittelkettige Triglyceride) oder natürliche Öle, wie Soja-, Erdnuss-, Avocado-, Oliven-, Rizinusöl, sowie Nuss- oder Samenöle.

Der mindestens eine Konservierungsstoff ist z.B. Paraben.

Das mindestens eine Oligonukleotid ist ausgewählt aus der Gruppe der DNAzyme

Beispielhaft aber nicht abschließend werden als DNAzyme die DNAzyme der DE 103 46 487.5 genannt, die gegen die mRNA der Proteine GATA-3 und T-bet gerichtet sind und die zur Herstellung eines Mittels gegen entzündliche Erkrankungen offenbart sind.

Beispielsweise werden folgende DNAzyme verwendet (jedes einzeln oder in Kombination mit den anderen):
Name der DNAzyme gegen GATA-3 mRNA-Sequenz:

| | |
|---|---|
| hgd1 | 5'-TCGGTCAGAggctagctacaacgaTGCGTTGCT-3' |
| hgd2 | 5'-GGCGTACGAggctagctacaacgaCTGCTCGGT-3' |
| hgd3 | 5'-GGCGGCGTAggctagctacaacgaGACCTGCTC-3' |
| hgd4 | 5'-CTCGGGTCAggctagctacaacgaCTGGGTAGC-3' |
| hgd5 | 5'-TCCTCTGCAggctagctacaacgaCGGGGTCCT-3' |
| hgd6 | 5'-ACTCTGCAAggctagctacaacgaTCTGCGAGC-3' |
| hgd7 | 5'-GGGCGACGAggctagctacaacgaTCTGCAATT-3' |
| hgd8 | 5'-AAGGGGCGAggctagctacaacgaGACTCTGCA-3' |
| hgd9 | 5'-AAAACGGGAggctagctacaacgaCAGGTTGTA-3' |
| hgd10 | 5'-AGAATAAAAggctagctacaacgaGGGACCAGG-3' |
| hgd11 | 5'-ATGGCAGAAggctagctacaacgaAAAACGGGA-3' |
| hgd12 | 5'-AACTGGGTAggctagctacaacgaGGCAGAATA-3' |
| hgd13 | 5'-ATCCAAAAAggctagctacaacgaTGGGTATGG-3' |
| hgd14 | 5'-AGGGGAAGAggctagctacaacgaAAAAATCCA-3' |
| hgd15 | 5'-TTTTAAAAAggctagctacaacgaTATCTTGGA-3' |
| hgdl6 | 5'-GTGGGGGGAggctagctacaacgaGGGAAGGCT-3' |
| hgdl7 | 5'-GTTGAATGAggctagctacaacgaTTGCTTTCG-3' |
| hgdl8 | 5'-GTCGTTGAAggctagctacaacgaGATTTGCTT-3' |
| hgd19 | 5'-GGCCCGGAAggctagctacaacgaCCGCGCGCG-3' |
| hgd20 | 5'-TCACCTCCAggctagctacaacgaGGCCTCGGC-3' |
| hgd21 | 5'-CCGCCGTCAggctagctacaacgaCTCCATGGC-3' |
| hgd22 | 5'-GGTGGCTCAggctagctacaacgaCCAGCGCGG-3' |
| hgd23 | 5'-CGTTGAGCAggctagctacaacgaGGCGGGGTG-3' |
| hgd24 | 5'-CCGCGTCCAggctagctacaacgaGTAGGAGTG-3' |
| hgd25 | 5'-CAGCGGGTAggctagctacaacgaTGCGCCGCG-3' |
| hgd26 | 5'-GCACATCCAggctagctacaacgaCTCCTCCGG-3' |
| hgd27 | 5'-AAAAGCACAggctagctacaacgaCCACCTCCT-3' |
| hgd28 | 5'-TAAAAAGCAggctagctacaacgaATCCACCTC-3' |
| hgd29 | 5'-GACCGTCGAggctagctacaacgaGTTAAAAAG-3' |
| hgd30 | 5'-TTGCCTTGAggctagctacaacgaCGTCGATGT-3' |
| hgd31 | 5'-AGGGCGGGAggctagctacaacgaGTGGTTGCC-3' |
| hgd32 | 5'-TGGCCCTGAggctagctacaacgaCGAGTTTCC-3' |
| hgd33 | 5'-ACCTCTGCAggctagctacaacgaCGTGGCCCT-3' |
| hgd34 | 5'-CGGAGGGTAggctagctacaacgaCTCTGCACC-3' |
| hgd35 | 5'-GGCGGCACAggctagctacaacgaCTGGCTCCC-3' |
| hgd36 | 5'-CGGGCGGCAggctagctacaacgaACCTGGCTC-3' |
| hgd37 | 5'-AGGGATCCAggctagctacaacgaGAAGCAGAG-3' |
| hgd38 | 5'-GGGTAGGGAggctagctacaacgaCCATGAAGC-3' |
| hgd39 | 5'-GGGCTGAGAggctagctacaacgaTCCAGGGGG-3' |
| hgd40 | 5'-GTGGATGGAggctagctacaacgaGTCTTGGAG-3' |
| hgd41 | 5'-CGTGGTGGAggctagctacaacgaGGACGTCTT-3' |
| hgd42 | 5'-GGGGGTAGAggctagctacaacgaGGAGAGGGG-3' |
| hgd43 | 5'-GGAGGAGGAggctagctacaacgaGAGGCCGGG-3' |
| hgd44 | 5'-GCCCCCCGAggctagctacaacgaAAGGAGGAG-3' |
| hgd45 | 5'-CCGGGGAGAggctagctacaacgaGTCCTTCGG-3' |
| hgd46 | 5'-GGACAGCGAggctagctacaacgaGGGTCCGGG-3' |
| hgd47 | 5'-TGGGGTGGAggctagctacaacgaAGCGATGGG-3' |
| hgd48 | 5'-CTTGAGGCAggctagctacaacgaTCTTTCTCG-3' |
| hgd49 | 5'-CACCTGGTAggctagctacaacgaTTGAGGCAC-3' |
| hgd50 | 5'-GCAGGGGCAggctagctacaacgaCTGGTACTT-3' |
| hgd51 | 5'-CCAGCTTCAggctagctacaacgaGCTGTCGGG-3' |
| hgd52 | 5'-GTGGGACGAggctagctacaacgaTCCAGCTTC-3' |
| hgd53 | 5'-GGAGTGGGAggctagctacaacgaGACTCCAGC-3' |
| hgd54 | 5'-ATGCTGCCAggctagctacaacgaGGGAGTGGG-3' |
| hgd55 | 5'-GGGCGGTCAggctagctacaacgaGCTGCCACG-3' |
| hgd56 | 5'-GAGGCTCCAggctagctacaacgaCCAGGGCGG-3' |
| hgd57 | 5'-GTGGGTCGAggctagctacaacgaGAGGAGGCT-3' |
| hgd58 | 5'-AGGTGGTGAggctagctacaacgaGGGGTGGTG-3' |
| hgd59 | 5'-ACTCGGGCAggctagctacaacgaGTAGGGCGG-3' |
| hgd60 | 5'-GGAGCTGTAggctagctacaacgaTCGGGCACG-3' |
| hgd61 | 5'-GGACTTGCAggctagctacaacgaCCGAAGCCG-3' |
| hgd62 | 5'-GGGCCTGGAggctagctacaacgaTTGCATCCG-3' |
| hgd63 | 5'-TGTGCTGGAggctagctacaacgaCGGGCCTTG-3' |
| hgd64 | 5'-GTTCACACAggctagctacaacgaTCCCTGCCT-3' |
| hgd65 | 5'-CAGTTCACAggctagctacaacgaACTCCCTGC-3' |
| hgd66 | 5'-CACAGTTCAggctagctacaacgaACACTCCCT-3' |
| hgd67 | 5'-GTTGCCCCAggctagctacaacgaAGTTCACAC-3' |
| hgd68 | 5'-TCGCCGCCAggctagctacaacgaAGTGGGGTC-3' |
| hgd69 | 5'-CCCGTGCCAggctagctacaacgaCTCGCCGCC-3' |
| hgd70 | 5'-GGCGTTGCAggctagctacaacgaAGGTAGTGT-3' |

Name der DNAzyme gegen T-bet mRNASequenz:

| | |
|---|---|
| td1 | 5'-TGGCTTCTAggctagctacaacgaGCCCTCGTC-3' |
| td2 | 5'-GGGCTCTGAggctagctacaacgaGCCTGGCTT-3' |
| td3 | 5'-GGGACCCCAggctagctacaacgaCGGAGCCCG-3' |
| td4 | 5'-GGTGGGGGAggctagctacaacgaCCCACCGGA-3' |
| td5 | 5'-GGCGGGGGAggctagctacaacgaCCGAGGGCC-3' |
| td6 | 5'-GGGCTGGGAggctagctacaacgaGGGCAGGGA-3' |
| td7 | 5'-CGTCGAGGAggctagctacaacgaCCGCCCCTC-3' |
| td8 | 5'-GGGCTGGCAggctagctacaacgaCTTCCCGTA-3' |
| td9 | 5'-CGATGCCCAggctagctacaacgaCCGGGGCGG-3' |
| td10 | 5'-GCTCCACGAggctagctacaacgaGCCCATCCG-3' |
| td11 | 5'-CCGGCTCCAggctagctacaacgaGATGCCCAT-3' |
| td12 | 5'-TCTCCGCAAggctagctacaacgaCCGGCTCCA-3' |
| td13 | 5'-CCGTCAGCAggctagctacaacgaGTCTCCGCA-3' |
| td14 | 5'-TCCCCGGCAggctagctacaacgaCGGCTCGGT-3' |
| td15 | 5'-CCCCCGCGAggctagctacaacgaGCTCGTCCG-3' |
| td16 | 5'-GTAGGGAGAggctagctacaacgaCCCAGGCTG-3' |
| td17 | 5'-GGGCGGGCAggctagctacaacgaCAAGGCGCC-3' |
| td18 | 5'-CGGGAAGGAggctagctacaacgaTCGCCCGCG-3' |
| td19 | 5'-TAGTCCTCAggctagctacaacgaGCGGCCCCG-3' |
| td20 | 5'-TCCCCGACAggctagctacaacgaCTCCAGTCC-3' |
| td21 | 5'-TTTCCCCGAggctagctacaacgaACCTCCAGT-3' |
| td22 | 5'-TGAGCGCGAggctagctacaacgaCCTCAGTTT-3' |
| td23 | 5'-GGACCACAAggctagctacaacgaAGGTGGTTG-3' |
| td24 | 5'-CTTGGACCAggctagctacaacgaAACAGGTGG-3' |
| td25 | 5'-AAACTTGGAggctagctacaacgaCACAACAGG-3' |
| td26 | 5'-CTGATTAAAggctagctacaacgaTTGGACCAC-3' |
| td27 | 5'-TGGTGCTGAggctagctacaacgaTAAACTTGG-3' |
| td28 | 5'-TGATGATCAggctagctacaacgaCTCTGTCTG-3' |
| td29 | 5'-TGGTGATGAggctagctacaacgaCATCTCTGT-3' |
| td30 | 5'-GCTTGGTGAggctagctacaacgaGATCATCTC-3' |
| td31 | 5'-ATGGGAACAggctagctacaacgaCCGCCGTCC-3' |
| td32 | 5'-GAATGGGAAggctagctacaacgaATCCGCCGT-3' |
| td33 | 5'-TGACAGGAAggctagctacaacgaGGGAACATC-3' |
| td34 | 5'-AGTAAATGAggctagctacaacgaAGGAATGGG-3' |
| td35 | 5'-CACAGTAAAggctagctacaacgaGACAGGAAT-3' |
| td36 | 5'-GCCCGGCCAggctagctacaacgaAGTAAATGA-3' |
| td37 | 5'-CCACAAACAggctagctacaacgaCCTGTAGTG-3' |
| td38 | 5'-GTCCACAAAggctagctacaacgaATCCTGTAG-3' |
| td39 | 5'-CCACGTCCAggctagctacaacgaAAACATCCT-3' |
| td40 | 5'-CCAAGACCAggctagctacaacgaGTCCACAAA-3' |
| td41 | 5'-CCACCAAGAggctagctacaacgaCACGTCCAC-3' |
| td42 | 5'-GCTGGTCCAggctagctacaacgaCAAGACCAC-3' |
| td43 | 5'-GCTCTGGTAggctagctacaacgaCGCCAGTGG-3' |
| td44 | 5'-CTGCACCCAggctagctacaacgaTTGCCGCTC-3' |
| td45 | 5'-CACACTGCAggctagctacaacgaCCACTTGCC-3' |
| td46 | 5'-CTTTCCACAggctagctacaacgaTGCACCCAC-3' |
| td47 | 5'-GCCTTTCCAggctagctacaacgaACTGCACCC-3' |
| td48 | 5'-TTCCTGGCAggctagctacaacgaGCTGCCCTC-3' |
| td49 | 5'-GTGGACGTAggctagctacaacgaAGGCGGTTT-3' |
| td50 | 5'-CCGGGTGGAggctagctacaacgaGTACAGGCG-3' |
| td51 | 5'-CCTGGCGCAggctagctacaacgaCCAGTGCGC-3' |
| td52 | 5'-CAAATGAAAggctagctacaacgaTTCCTGGCG-3' |
| td53 | 5'-TTTCCCAAAggctagctacaacgaGAAACTTCC-3' |
| td54 | 5'-ATTGTTGGAggctagctacaacgaGCCCCCTTG-3' |
| td55 | 5'-TGGGTCACAggctagctacaacgaTGTTGGACG-3' |
| td56 | 5'-TCTGGGTCAggctagctacaacgaATTGTTGGA-3' |
| td57 | 5'-GCACAATCAggctagctacaacgaCTGGGTCAC-3' |
| td58 | 5'-GGAGCACAAggctagctacaacgaCATCTGGGT-3' |
| td59 | 5'-ACTGGAGCAggctagctacaacgaAATCATCTG-3' |
| td60 | 5'-ATGGAGGGAggctagctacaacgaTGGAGCACA-3' |
| td61 | 5'-TGGTACTTAggctagctacaacgaGGAGGGACT-3' |
| td62 | 5'-GGGCTGGTAggctagctacaacgaTTATGGAGG-3' |
| td63 | 5'-TCAACGATAggctagctacaacgaGCAGCCGGG-3' |
| td64 | 5'-CCTCAACGAggctagctacaacgaATGCAGCCG-3' |
| td65 | 5'-TCACCTCAAggctagctacaacgaGATATGCAG-3' |
| td66 | 5'-CGTCGTTCAggctagctacaacgaCTCAACGAT-3' |
| td67 | 5'-GTAAAGATAggctagctacaacgaGCGTGTTGG-3' |
| td68 | 5'-AAGTAAAGAggctagctacaacgaATGCGTGTT-3' |
| td69 | 5'-GGCAATGAAggctagctacaacgaTGGGTTTCT-3' |
| td70 | 5'-TCACGGCAAggctagctacaacgaGAACTGGGT-3' |
| td71 | 5'-AGGCAGTCAggctagctacaacgaGGCAATGAA-3' |
| td72 | 5'-ATCTCGGCAggctagctacaacgaTCTGGTAGG-3' |
| td73 | 5'-GCTGAGTAAggctagctacaacgaCTCGGCATT-3' |
| td74 | 5'-TATTATCAAggctagctacaacgaTTTCAGCTG-3' |
| td75 | 5'-GGGTTATTAggctagctacaacgaCAATTTTCA-3' |
| td76 | 5'-AAGGGGTTAggctagctacaacgaTATCAATTT-3' |
| td77 | 5'-CTCCCGGAAggctagctacaacgaCCTTTGGCA-3' |
| td78 | 5'-GTACATGGAggctagctacaacgaTCAAAGTTC-3' |

### Bestandteile der W-O-W Emulsion:

Die W-O-W Emulsion umfasst mindestens einen lipophilen Emulgator, mindestens eine amphiphile Komponente, mindestens einen Konsistenzgeber, mindestens eine okklusive Komponente, mindestens einen anorganischen und organischen Zusatz, mindestens einen hydrophilen Emulgator.

Insbesondere umfasst die die W-O-W Emulsion mindestens einen lipophilen Emulgator, wobei der lipophile Emulgator ausgewählt ist aus der Gruppe Sorbitan Fettsäureester, Glycerol-Derivate (z.B. Spans).

Mindestens eine amphiphile Komponente, wobei diese amphiphile Komponente ausgewählt ist aus der Gruppe Phospholipide, vorzugsweise Lecithin.

Mindestens einen Konsistenzgeber, wobei der Konsistenzgeber ausgewählt ist aus der Gruppe Fettsäureester (z.B. Cetylpalmitat, Myristylmyristate) Polyethylenglycole, Cera Alba, mikrokristallines Wachs, Wollwachs, Hydrogenated Castor Oil, Protegin W, Protegin WX, Carbomere (z.B. quervernetzte Acrylsäurepolymere), oder Cellulose.

Mindestens eine okklusive Komponente, wobei die okklusive Komponente ein gesättigter Kohlenwasserstoff ist ausgewählt aus der Gruppe Paraffin oder Polysiloxane, wie Silikonöle.

Alternativ ein Öl, ausgewählt aus der Gruppe der veresterten Fettsäuren (z.B. Decyl oleate), Wachse (z.B. Jojobawax) oder partiell veresterte Glyceride (z.B. mittelkettige Triglyceride) oder natürliche Öle, wie Soja-, Erdnuss-, Avocado-, Oliven-, Rizinusöl, sowie Nuss- oder Samenöle.

Mindestens einen anorganischen und/oder organischen Zusatz, bevorzugt ein Salz oder eine ionische Flüssigkeit dessen Kationenanteil Na, Mg, K, Li, Ca, Fe, Cu Ag oder eine Kombination der Elemente umfasst.

Mindestens einen hydrophilen Emulgator, wobei der hydrophile Emulgator ausgewählt ist aus der Gruppe Polysorbate, ethoxylierte Polyethylenglycole (wie z.B. Tweens, Steareths, Laureths, Ceteareths), etoxylierte Ether, etoxylierte. Ester.

Mindestens ein Oligonukleotid ausgewählt aus der Gruppe Antisense-Oligonukleotide, wie z.B. DNAzyme, siRNAs, asDNAs oder Ribozyme,.oder Primer oder Aptamere.

Alternativ umfasst die W-O-W Emulsion zusätzlich mindestens einen Feuchthaltefaktor, wobei der Feuchthaltefaktor ausgewählt ist aus der Gruppe Glycerin, Polyole, Osmolyte.

Alternativ umfasst die W-O-W Emulsion zusätzlich mindestens einen Konservierungsstoff auf, wie z.B. Paraben.

### Bestandteile der W-O Emulsion:

Die W-O Emulsion umfasst mindestens einen lipophilen Emulgator, mindestens eine okklusive Komponente, mindestens einen Konsistenzgeber, mindestens einen anorganischen und/oder organischen Zusatz, mindestens einen hydrophilen Emulgator.

Insbesondere umfasst die die W-O Emulsion mindestens einen lipophilen Emulgator, wobei der lipophile Emulgator ausgewählt ist aus der Gruppe Sorbitan Fettsäureester und Glycerol-Derivate, wobei die Glycerol-Derivate ausgewählt sind aus der Gruppe bestehend aus Glycerol Stearate, Glycerol dioleate, Glycerolmonooleate.

Mindestens eine okklusive Komponente, wobei die okklusive Komponente ein gesättigter Kohlenwasserstoff ist ausgewählt aus der Gruppe Paraffin oder Polysiloxane, wie Silikonöle.

Alternativ ein Öl, ausgewählt aus der Gruppe der veresterten Fettsäuren (z.B. Decyl oleate), Wachse (z.B. Jojobawax) oder partiell veresterte Glyceride (z.B. mittelkettige Triglyceride) oder natürliche Öle, wie Soja-, Erdnuss-, Avocado-, Oliven-, Rizinusöl, sowie Nuss- oder Samenöle.

Mindestens einen Konsistenzgeber, wobei der Konsistenzgeber ausgewählt ist aus der Gruppe Fettsäureester (z.B. Cetylpalmitat, Myristylmyristate) Polyethylenglycole, Cera Alba, mikrokristallines Wachs, Wollwachs oder seine Alkohole, Hydrogenated Castor Oil, Protegin W, Protegin WX, Carbomere (z.B. quervernetzte Acrylsäurepolymere), oder Cellulose.

Mindestens einen anorganischen und oder organischen Zusatz, bevorzugt ein Salz oder eine ionische Flüssigkeit dessen Kationenanteil Na, Mg, K, Li, Ca, Fe, Cu Ag oder eine Kombination der Elemente umfasst.

Alternativ umfasst die W-O Emulsion zusätzlich mindestens einen Feuchthaltefaktor, wobei der Feuchthaltefaktor ausgewählt ist aus der Gruppe Glycerin, Polyole, Osmolyte.

Mindestens ein Oligonukleotid ausgewählt aus der Gruppe Antisense-Oligonukleotide, wie z.B. DNAzyme, siRNAs, asDNAs oder Ribozyme,.oder Primer oder Aptamere.

Alternativ umfasst die W-O Emulsion zusätzlich mindestens einen Konservierungsstoff auf, wie z.B. Paraben.

### Herstellung der Emulsionen

Die erfindungsgemäße pharmazeutische Zusammensetzung zur topischen Anwendung umfassend, mindestens einen lipophilen Emulgator, mindestens einen Konsistenzgeber, mindestens eine okklusive Komponente, mindestens einen anorganischen und/oder organischen Zusatz und mindestens ein Oligonukleotid wird/ werden nach einem dem Fachmann geläufigen Verfahren zusammengegeben.

Die erfindungsgemäße pharmazeutische Zusammensetzung zur topischen Anwendung umfassend, mindestens einen lipophilen Emulgator, mindestens einen Konsistenzgeber, mindestens eine okklusive Komponente, mindestens einen anorganischen und/oder organischen Zusatz und mindestens ein Oligonukleotid ist
mehr oder weniger fluide, ist ein Shampoo, eine Lösung, eine Lotion, Creme, Salbe, Milch, eine Paste oder ein Schaum. Sie ist alternativ ein Aerosol und wird über die Lunge appliziert.

Die erfindungsgemäße pharmazeutische Zusammensetzung zur topischen Anwendung umfassend, mindestens einen lipophilen Emulgator, mindestens einen Konsistenzgeber, mindestens eine okklusive Komponente, mindestens einen anorganischen und/oder organischen Zusatz und mindestens ein Oligonukleotid wird vor allem bei Säugern insbesondere bei Menschen verwendet.

### 2. Verwendung als Mittel zur Behandlung und Prophylaxe von Erkrankungen

Die erfindungsgemäße Formulierung in Form einer Emulsion mit dispergierter, innenliegender, diskontinuierlicher Wasserphase ist durch den Zusatz von Magnesium- oder Natrium-Ionen besonders zum Schutz der Oligonukleotide geeignet, da diese stabilisiert und vor enzymatischem Abbau geschützt und eine gute Aufnahme in die Zielzellen ermöglicht wird. Daher eignet sich die erfindungsgemäße Formulierung als
kosmetische und/oder dermatologische und/oder pharmazeutische Zubereitung für die topische Anwendung und Applikation. Durch die Nutzung von Oligonukleotiden, die gegen Entzündungserkrankungen wirksam bekannt sind, z.B. die DNAzyme der DE 103 46 487.5 eignet sich die erfindungsgemäße Formulierung zur Herstellung eines Mittels zu Therapie und Prophylaxe entzündlicher Erkrankungen, das zur topischen Anwendung verwendet wird.

Die erfindungsgemäße Formulierung zeigt eine gute Aufnahme der Oligonukleotide z.B. der DNAzyme insbesondere der DNAzyme der DE 103 46 487.5 in Zielzellen der topischen Anwendung z.B. Hautzellen, Lungen- und Darmepithelzellen, Schleimhaut, Nasenepithelzellen und Zellen des Rachenraums.

### 3. Ausführungsbeispiele

### 3.1. Ausführungsbeispiel für WOW-Emulsion

Die erfindungsgemäßen DNAzym-haltigen Wasser-in-Öl-in-Wasser Emulsionen (W-O-W-Emulsionen) umfassen bevorzugt folgende Bestandteile:

| | **W-O-W** | [%] (Bereich |
|---|---|---|
| Lipophiler Emulgator, vorzugsweise Sorbitan Fettsäureester, Glycerol-Derivate (z.B. Spans) | | 0,5-20 |
| Amphiphile Komponente, Phospholipide, vorzugsweise Lecithin, | | 0,05-5 |
| Konsistenzgeber, wie Fettsäureester (z.B. Cetylpalmitat, Myristylmyristate) Polyethylenglycole, Cera Alba, mikrokristallines Wachs, Wollwachs, Hydrogenated Castor Oil, Carbomere (z.B. quervernetzte Acrylsäurepolimere), Protegin W, Protegin WX oder Cellulose | | 0,1-5 |
| Okklusive Komponente, auf Basis gesättigter Kohlenwasserstoffe, wie Paraffin oder Polysiloxane, wie Silikonöle | | 1-25 |
| Alternativ ein Öl, ausgewählt aus der Gruppe der veresterten Fettsäuren (z.B. Decyl oleate), Wachse (z.B. Jojobawax) oder partiell veresterte Glyceride (z.B. mittelkettige Triglyceride) oder natürliche Öle, wie Soja-, Erdnuss-, Avocado-, Oliven-, Rizinusöl, sowie Nuss- oder Samenöle. | | 1-25 |

| | | |
|---|---|---|
| Anorganischer und oder organischer Zusatz, z.B. ein Salz oder ionische Flüssigkeit deren Kationenanteil Na, Mg, K, Li, Ca, Fe, Cu, Ag oder eine Kombination der Elemente umfasst | | 0,01-2 |
| Oligonukleotid z.B. eines der DNAzyme der DE 103 46 487.5 | | 0,01-5 |
| Wasser | | q.s. |
| Hydrophiler Emulgator, wie Polysorbate oder ethoxylierte Polyethylenglycole (z.B. Tweens, Steareths, Laureths, Ceteareths) oder etoxylierte Ether bzw. Ester | | 1-8 |
| Feuchthaltefaktoren (z.B. Glycerin, Polyole, Osmolyte) | | 0,1-10 |
| Konservierungsstoffe, wie Parabene | | q.s. |

### 3. 1. 1. Ausgewähltes Ausführungsbeispiel (W-O-W 167)

| **W-O-W 167** | [% w/w] |
|---|---|
| Sorbitan Monooleate | 4 |
| Lecithin | 0,2 |
| Parafin | 15,8 |
| NaCl | 0,074 |
| DNAzym hdg 40 | 0,4 |
| Wasser | ad. 100 |
| Steareth-20 | 1 |
| Wasser+Parabene | 59 |

### 3. 1. 2. Ausgewähltes Ausführungsbeispiel (W-O-W 146)

| | **W-O-W 146** | [% w/w] |
|---|---|---|
| Sorbitan Monooleate | | 4 |
| Lecithin | | 0,2 |
| Parafin | | 15,8 |
| MgSO₄ x 7 H₂O | | 0,308 |
| DNAzym hdg40 | | 0,4 |
| Wasser | | ad. 100 |
| Wasser+Parabenekonserviertes | | 59 |
| Wasser (äußere Phase) | | |

### 3.2. Ausführungsbeispiel für WO-Emulsion

Die erfindungsgemäßen DNAzym-haltigen Wasser-in-Öl Emulsionen (W-O-Emulsionen) umfassen bevorzugt folgende Bestandteile:

| | **W-O** | [%] Bereich |
|---|---|---|
| Ein oder Mischung aus lipopihlen Emulgatoren, wie Sorbitan Fettsäureester oder Glycerol-Derivate (Glycerol Stearate, Glycerol dioleate, Glycerolmonooleate) | | 1-15 |
| | Okklusive Komponente, auf Basis gesättigter Kohlenwasserstoffe, wie Paraffin oder Polysiloxane, wie Silikonöle | 10-70 |
| | Alternativ ein Öl, ausgewählt aus der Gruppe der veresterten Fettsäuren (z.B. Decyl oleate), Wachse (z.B. Jojobawax) oder partiell veresterte Glyceride (z.B. mittelkettige Triglyceride) oder natürliche Öle, wie Soja-, Erdnuss-, Avocado-, Oliven-, Rizinusöl, sowie Nuss- oder Samenöle. | 10-70 |
| | Konsistenzgeber, wie Fettsäureester (z.B. Cetylpalmitat, Myristylmyristate) Polyethylenglycole, Cera Alba, mikrokristallines Wachs, Wollwachs, Hydrogenated Castor Oil, ProteginW, Protegin WX, Carbomere (z.B. quervernetzte Acrylsäurepolymere), oder Cellulose | 0,5-10 |
| Wollwachs oder seine Alkohole | | 1-8 |
| Konservierungsstoffe (z.B. Parabene) | | q.s. |
| Anorganischer und oder organischer Zusatz, z.B. ein Salz oder ionische Flüssigkeit deren Kationenanteil Na, Mg, K, Li, Ca, Fe, Cu, Ag oder eine Kombination der Elemente umfasst | | 0,01-2 |
| Oligonukleotid z.B. eines der DNAzyme der DE 103 46 487.5 | | 0,01-5 |
| Feuchthaltefaktoren (z.B. Glycerin, Polyole, Osmolyte) | | 0,1-10 |

### 3. 2.1. Ausgewähltes Ausführungsbeispiel (W-O 126)

| | **W-O 126** | [% w/w] |
|---|---|---|
| Glycerol Stearate | | 1 |
| Glycerol mono oleate (Imwitor 946) | | 2 |
| Glycerol dioleate (Crossential GDO) | | 2 |
| Paraffin | | 38 |
| Cethylpalmitat | | 2 |
| Lanolin | | 3 |
| Hydrogenated Castor Oil | | 2 |
| Konserviertes Wasser+Parabene | | ad. 100 |
| NaCl | | 0,5 |
| DNAzym hdg40 | | 0,4 |
| Glycerin | | 3 |

### 3. 2.2. Ausgewähltes Ausführungsbeispiel (W-O 162)

| | **W-O 162** | [% w/w] |
|---|---|---|
| Glycerol Stearate | | 1 |
| Glycerol mono oleate (Imwitor 946) | | 2 |
| Glycerol dioleate (Crossential GDO) | | 2 |
| Paraffin | | 38 |
| Cethylpalmitat | | 2 |
| Lanolin | | 3 |
| Wasser+Parabenekonserviertes Wasser | | ad. 100 |
| MgSO₄ x 7 H₂O | | 1 |
| DNAzym hdg 40 | | 0,4 |
| Glycerin | | 3 |

Als Oligonukleotid werden beispielsweise alle in der DE 103 46 487.5 offenbarten DNAzyme hgd 1-70 und td 1-78 verwendet, bevorzugt wird hgd 40 als gegen GATA-3 gerichtetes DNAzym verwendet und td69 bzw. td70 als gegen T-bet gerichtete DNAzyme.

### 4. Stabilitätsmessungen der Formulierungen

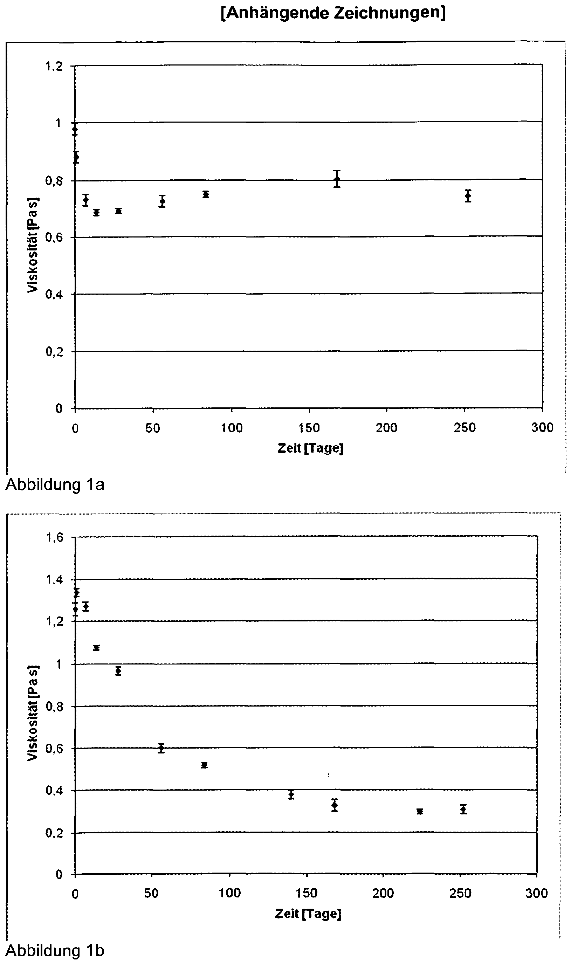

Die erfindungsgemäßen DNAzym-haltigen Formulierungen WOW 167 und WOW 146 unterscheiden sich durch die zugesetzten Elektrolyte in der inneren Wasserphase, grafisch dargestellt in Abbildung 1. Dabei werden für die Herstellung von WOW 167 und WOW 146 bevorzugt Lösungen mit 0,13 M verwendet, Formulierungen mit höheren Konzentrationen an Elektrolyten verringern die Stabilität der Formulierungen.

Die Messung der Stabilität erfolgt im Vergleich und über einen Zeitraum von 150 Tagen.

Direkt nach der Formulierung ist die Viskosität der WOW 167 (Formulierung mit NaCl) mit 1,4 Pa s etwas höher, als die Viskosität der WOW 146 (Formulierung mit MgSO₄) mit 1 Pa s. Die Viskosität der Formulierung WOW 146 (Formulierung mit MgSO₄) sinkt in den ersten 5 Tagen etwas ab auf 0,75 Pa s, bleibt danach weitgehend unverändert bei 0,75 Pa s. Die Viskosität der WOW 167 (Formulierung mit NaCl) sinkt bis zum Tag 50 auf 0,6 Pa s und dann weiter auf 0,4 Pa s ab.

Die WOW 146 (Formulierung mit MgSO₄) ist somit deutlich stabiler als die WOW 167 (Formulierung mit NaCl). Anzumerken ist, dass der leichte Abfall der Viskositäten sich nicht negativ auf die Stabilität oder den Schutz auswirkt, die Emulsionen zeigen keinen Phasenumschlag oder Separation und sind stabil.

Die gemessenen Tropfengrößen liegen im Bereich von 10-20 µm.

Die erfindungsgemäßen DNAzym-haltigen Formulierungen WO 162 und WO 126 unterscheiden sich ebenfalls durch die zugesetzten Elektrolyte in der inneren Wasserphase. Die Messung der Stabilität erfolgt im Vergleich und über einen Zeitraum von 150 Tagen, wobei die Viskosität über 150 Tage unverändert bei etwa 4 Pa s für WO 126 und 4,2 für WO 162 liegt.

Die Tropfengröße liegt hier im Bereich von 0,5- 2,5 µm.

### 5. Stabilität gegenüber dem Abbau durch Nukleasen z.B. DNAsen

Die erfindungsgemäßen DNAzym-haltigen Formulierungen WOW 167 und WOW 146 enthalten die DNAzyme der DE 103 46 487.5, jeweils DNAzyme hgd 1 bis hgd 70 gegen GATA-3 und die DNAzyme td 1 bis td 78 gegen T-bet und schützen diese gegen den Abbau bzw. die Degradation durch Nukleasen insbesondere DNAsen. Es ist wichtig die DNAzyme vor dem DNAse Abbau zu schützen, damit sie ihre therapeutische Wirksamkeit entfalten können. Experimentell wird zur Messung der Stabilität der erfindungsgemäßen DNAzym- enthaltenden Formulierungen gegenüber DNAse eine kommerzielle erhältliche DNAse I mit einer Aktivität von 105 U verwendet und den Formulierungen WOW 167 und WOW 146 zugesetzt. Des Weiteren wird der Abbau durch ein Hautlysat als Positivkontrolle gezeigt. Der Abbau der DNAzyme wird mittels HPLC beobachtet.

Diese Messung erfolgt mit folgender Methode:

### Abbau von DNAzyme durch DNAse aus der Haut

### 1) Verfahren

Haut: ca. 50 mg
Lösung: 1 ml DNAzym-Standard (0,1625 mg/ml) + 3 ml RO-Wasser (Wasser aus Umkehrosmose)
Verfahren: Haut mit Skalpell zerkleinert und mit Lösung versetzt und leicht geschüttelt. Danach filtriert und Abbau mittels HPLC nach 2min, 52min, 102min und 152min untersucht.

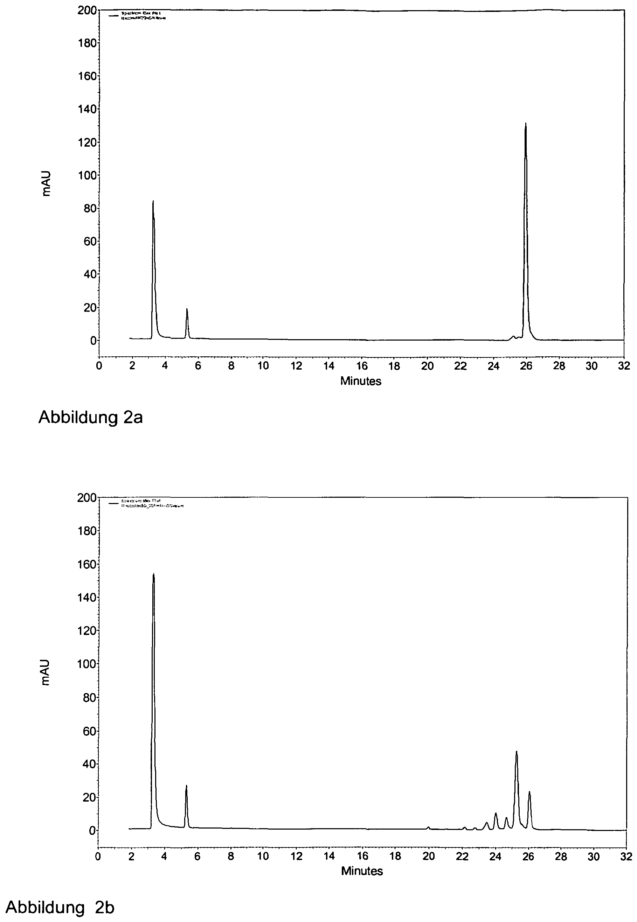

Die Abbildungen 2a und 2b zeigen, dass die natürlich in der Haut vorkommenden DNAsen das DNAzym bereits nach 2 Minuten beginnen abzubauen, nach 152 Minuten ist nahezu die gesamte DNAzymmenge abgebaut.

### 6. Schutz des Abbaus von DNAzyme durch DNAse durch die entwickelten erfindungsgemäßen Formulierungen WOW 146, WOW 167, WO 126, WO 162

Galenik: 20 mg
Lösung: DNAse I mit Aktivität 105 U in Tris Puffer + 10mM MgSO₄
Verfahren: 20 mg der jeweiligen Formulierungen werden mit 1ml einer DNAse I- Lösung vermischt. Nach der Inkubationszeit von 1min wird das Gemisch für 10min in einen Thermomixer bei einer Temperatur von 99°C leicht geschüttelt, um die Aktivität der DNAse zu stoppen. Zum Aufbruch der Emulsion wird der Ansatz in ein Ultraschallbad bei 50°C 10 min inkubiert. Ansatz wird durch einen 0,45 µm Spritzenfilter filtriert und HPLC Analyse durchgeführt.

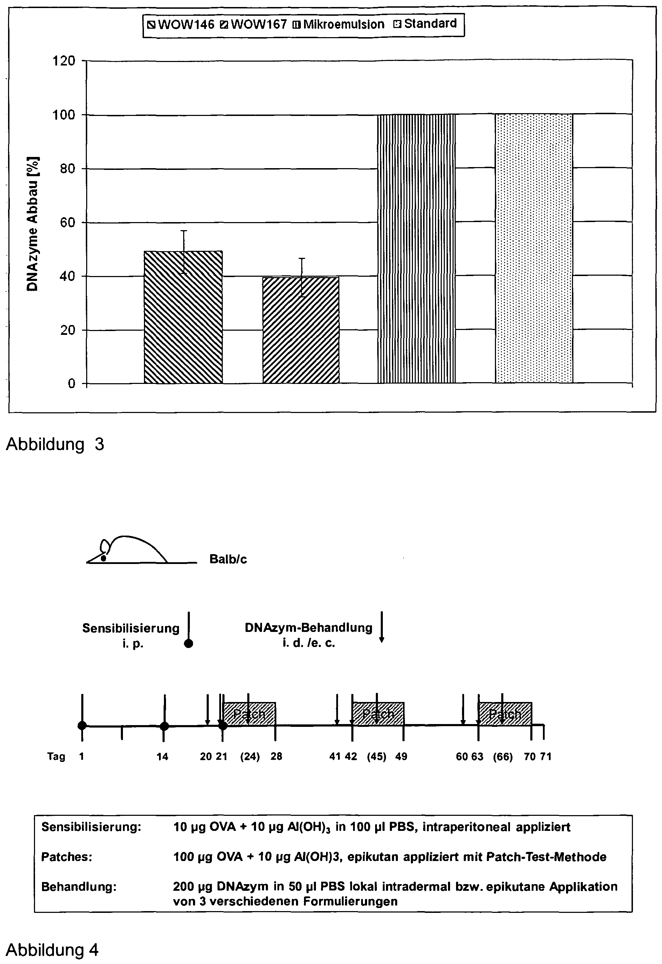

Abbildung 3 zeigt, dass die Formulierung WOW 146 einen Schutz der DNAzyme vor DNAse Abbau von etwa 61% aufweist, die Formulierung WOW 167 liegt bei einem einen Schutz von 51%. Als Kontrolle wurde eine DNAzyme-haltige Standardlösung (0,4% DNAzym hgd 40 in PBS-Puffer (PBS = phosphatgepufferte Salzlösung - 137mM NaCl, 2,7 mM KCI, 12mM Na₂HPO₄ und KH₂PO₄ pH=7,4)) eingesetzt, hier konnte kein Wirkstoff wiedergefunden werden. Somit schützt die WOW167 Formulierung insgesamt besser. Eine O/W-Vergleichsgalenik (Mikroemulsion: konserviertes Wasser, Ölkomponente, Glycerin, hydrophiler Emulgator, Magnesiumsulfat) mit gleicher Menge an DNAzyme zeigte ebenfalls keinen Schutz, es wurden 100% der DNAzyme abgebaut.

### 7. Stabilität im Vergleich zu ÖI-in-Wasser-Formulierungen (O-W-Formulierungen)

Zum Nachweis der Stabilität des Wirkstoffes in den Galeniken wurde die Wiederfindung von 0.4% des Wirkstoffes DNAzym hgd 40 in der WO 162, der W/OW 146 und als Referenz in einer O/W-Vergleichsgalenik (Mikroemulsion: konserviertes Wasser, Ölkomponente, Glycerin, hydrophiler Emulgator, Magnesiumsulfat) nach einem Monat mittels HPLC analysiert.

Die Wiederfindung an DNAzym hgd 40 gegenüber dem Startwert von 100% nach einem Monat beträgt 35,98 +/- 0,16% in der O/W-Vergleichsgalenik. In der W/O 162 liegt die Wiederfindung nach einem Monat bei 95,66 +/- 2,77%. Bei der W/O/W liegt die Wiederfindung nach einem Monat bei 103,15 +/- 2,29 %.

### 8. Nachweis der Wirksamkeit von GATA-3-DNAzym Formulierungen im Tiermodell

Die Wirksamkeit der erfindungsgemäßen pharmazeutischen Zusammensetzung zur topischen Anwendung umfassend, mindestens einen lipophilen Emulgator, mindestens einen Konsistenzgeber, mindestens eine okklusive Komponente, mindestens einen anorganischen und/oder organischen Zusatz und mindestens ein Oligonukleotid als Mittel zu Behandlung von entzündlichen Hauterkrankungen wird am Beispiel GATA-3 DNAzym-haltiger Formulierungen im Tiermodell dargestellt.

Dazu wird ein Mausmodell eingesetzt (siehe Abbildung 4), in dem durch allergische Sensibilisierung mit einem Modellallergen z.B. Ovalbumin (OVA) und nachfolgender wiederholter epikutaner Applikation über Hautpatches eine spezifische Entzündungsreaktion in der Haut der Mäuse induziert wird. Neben den typischen histologischen Veränderungen in der Haut wird dabei auch die erwartete erhöhte Expression des Zielgens z.B. GATA-3 sowie der Th2 Zytokine IL-4, IL-5 und IL-13 in den entzündeten Hautarealen ausgelöst und nachgewiesen. Durch Einsatz der erfindungsgemäßen DNAzym-haltigen Formulierungen als Mittel zu Behandlung von entzündlichen Hauterkrankungen wird eine therapeutische Wirksamkeit sichtbar. So wurde ein signifikant verminderter Entzündungs-Score in Mäusen nach Behandlung mit DNAzym-Formulierungen nachgewiesen. Zusätzlich wurde histologisch gezeigt, dass nach dieser Behandlung weniger Entzündungs-Zellen, insbesondere CD4+-T-Lymphozyten, in den entzündeten Hautarealen zu finden sind.

### Beispielsweise wurden eingesetzt

Sensibilisierung: 10 µg OVA + 10 µg Al(OH)₃ in 100 µl PBS, intraperitoneal appliziert
Patches: 100 µg OVA + 10 µg Al(OH)₃ epikutan appliziert mit Patch-test Methode
Behandlung: 200 µg DNAzym in 50 µl PBS lokal intradermal bzw. epikutan in den Formulierungen WOW146, WO162 und WOW167 appliziert
Die Auswertungen zeigen, dass die DNAzym-haltigen Formulierungen wirksam gegen experimentell erzeugte entzündliche Hauterkrankungen sind.

### [Abbildungslegenden und Bezugszeichenliste]

Abb. 1 zeigt die Viskosität [Pa*s] der DNAzym-haltigen Formulierungen Abb. 1a) WOW 146 (Formulierung mit MgSO₄) und Abb. 1b) 167 (Formulierung mit NaCl) im Vergleich über einen Zeitraum von 150 Tagen. Die Stabilität der WOW 146 (Formulierung mit MgSO₄) ist höher, als die Stabilität der WOW 167 (Formulierung mit NaCl).

Abbildung 2 zeigt den Abbau von DNAzym durch DNAse aus der Haut gemessen mit HPLC
Abbildung 2 a zeigt die HPLC-Messung von DNAzym auf Haut nach 2 min.
Abbildung 2 b zeigt die HPLC-Messung von DNAzym auf Haut nach 152 min.

Die Abbildungen 2a und 2b zeigen, dass der ungeschützte Abbau von DNAzmen auf Haut durch dort vorhandene DNAsen bereits nach 2 Minuten beginnt und nach 152 Minuten ist das DNAzym nahezu vollständig abgebaut ist. Diese Reaktion erfolgt mit allen Oligonukleotiden gleichermaßen und ist nicht auf DNAzyme eingeschränkt.

Abb. 3 zeigt den Schutz der DNAzym-haltigen Formulierungen gegenüber dem Abbau durch DNAse. Dargestellt ist die DNAzym-haltige Formulierungen WOW 146, die einen Schutz der DNAzyme über 90% aufweist, während die DNAzym-haltige Formulierung WOW 167 nur einen Schutz von 60% aufweist.

Als Kontrolle wird eine DNAzym-haltige Standardlösung (0,4 % DNAzym in PBS-Puffer) eingesetzt.

Abb. 4 zeigt den Versuchsaufbau eines Tiermodells, in dem experimentell eine entzündliche Hauterkrankungen induziert und die Wirksamkeit von Mitteln zur Behandlung dieser getestet wird.

Bei diesem Modell wird in der Haut von Mäusen (Stamm: Balb) durch allergische Sensibilisierung mit einem Modellallergen z.B. Ovalbumin (OVA) und nachfolgender wiederholter epikutaner Applikation über Hautpatches die für entzündliche Hauterkrankungen typische Entzündungsreaktion induziert. Die Sensibilisierung erfolgt durch intraperitoneale Applikation (i.p.), die Behandlung mit DNAzym durch intrademale (i.d.) Applikation oder epikutane (e.c.) Applikation verschiedener Formulierungen.

## Patentansprüche

1. Pharmazeutische Zusammensetzung zur topischen Anwendung umfassend,
- mindestens einen lipophilen Emulgator ausgewählt aus der Gruppe der Sorbitan Fettsäureester und Glycerol-Derivate, wobei die Glycerol-Derivate ausgewählt sind aus der Gruppe bestehend aus Glycerol Stearate, Glycerol dioleate und Glycerolmonooleate,
- einen hydrophilen Emulgator ausgewählt aus der Gruppe bestehend aus Polysorbate, ethoxylierte Polyethylenglycole, etoxylierte Ether und etoxylierte Ester,
- ein Phospholipid als amphiphile Komponente,
- mindestens einen Konsistenzgeber,
- mindestens einen gesättigten Kohlenwasserstoff ausgewählt aus der Gruppe Paraffin und Polysiloxane als okklusive Komponente oder alternativ ein synthetisches oder natürliches Öl oder Wax,
- mindestens einen anorganischen und/oder organischen Zusatz umfassend ein Salz oder eine ionische Flüssigkeit und
- mindestens ein Oligonukleotid,
**dadurch gekennzeichnet, dass**
- die pharmazeutische Zusammensetzung eine Wasser-in-ÖI-Wasser-(W-O-W)-Emulsion oder Wasser-in-Öl (W-O)-Emulsion mit dispergierter, innenliegender, diskontinuierlicher Wasserphase, unter Zusatz von Magnesium- oder Natriumionen, ist und das Oligonukleotid ein DNAzym ist, ausgewählt aus der Gruppe bestehend aus den DNAzymen hgd1 bis hgd70 und td1 bis td78.

2. Pharmazeutische Zusammensetzung gemäß Anspruch 1 umfassend mindestens einen Feuchthaltefaktor und/oder einen Konservierungsstoff.

3. Pharmazeutische Zusammensetzung gemäß Anspruch 1
**dadurch gekennzeichnet,**
**dass** das Salz einen Kationenanteil mit Na oder Mg, oder eine Kombination dieser Elemente umfasst.

4. Pharmazeutische Zusammensetzung gemäß Anspruch 3
**dadurch gekennzeichnet,**
**dass** die ionische Flüssigkeit eine Kombination der Elemente mit organischen Kationen umfasst.

5. Pharmazeutische Zusammensetzung gemäß Anspruch 1, **dadurch gekennzeichnet, dass** der mindestens eine hydrophile Emulgator ausgewählt ist aus der Gruppe bestehend aus Steareths, Laureths und Ceteareths.

6. Pharmazeutische Zusammensetzung gemäß Anspruch 1, **dadurch gekennzeichnet, dass** das Phospholipid der amphiphilen Komponente Lecithin ist.

7. Pharmazeutische Zusammensetzung gemäß Anspruch 1, **dadurch gekennzeichnet, dass** die Zusammensetzung mindestens einen Feuchthaltefaktor umfasst, wobei der mindestens eine Feuchthaltefaktor ausgewählt ist aus der Gruppe Glycerin, Polyole, Osmolyte.

## Claims

1. A pharmaceutical composition for topical application comprising,
- at least one lipophilic emulsifier selected from the group consisting of sorbitan fatty acid esters and glycerol derivatives, wherein the glycerol derivatives are selected from the group consisting of glycerol stearates, glycerol dioleates and glycerol monooleates,
- a hydrophilic emulsifier selected from the group consisting of polysorbates, ethoxylated polyethylene glycols, etoxylated ethers and etoxylated esters,
- a phospholipid as an amphiphilic component,
- at least one consistency enhancer,
- at least one saturated hydrocarbon selected from the group consisting of paraffin and polysiloxanes as occlusive component or alternatively a synthetic or natural oil or wax,
- at least one inorganic and/or organic additive comprising a salt or an ionic liquid,
and
- at least one oligonucleotide,
**characterized in that**
- the pharmaceutical composition is a water-in-oil-water (W-O-W) emulsion or a water-in-oil (W-O) emulsion with dispersed internal discontinuous water phase, with addition of magnesium or sodium ions, and the oligonucleotide is a DNAzyme selected from the group consisting of the DNAzymes hgd1 to hgd70 and td1 to td78.

2. A pharmaceutical composition according to claim 1 comprising at least one humectant and/or a preservative.

3. Pharmaceutical composition according to claim 1, **characterized in that** the salt comprises a cation part with Na or Mg, or a combination of these elements.

4. Pharmaceutical composition according to claim 3, **characterized in that** the ionic liquid comprises a combination of said elements with organic cations.

5. Pharmaceutical composition according to claim 1, **characterized in that** said at least one hydrophilic emulsifier is selected from the group consisting of steareths, laureths and ceteareths.

6. Pharmaceutical composition according to claim 1, **characterized in that** the phospholipid of the amphiphilic component is lecithin.

7. Pharmaceutical composition according to claim 1, **characterized in that** the composition comprises at least one humectant, wherein the at least one humectant is selected from the group consisting of glycerol, polyols, osmolytes.

## Revendications

1. Composition pharmaceutique pour application topique comprenant,
- au moins un émulsifiant lipophile choisi dans le groupe des esters d'acides gras du sorbitan et des dérivés du glycérol, les dérivés du glycérol étant choisis dans le groupe constitué par les stéarates de glycérol, les dioléates de glycérol et les monooléates de glycérol,
- un émulsifiant hydrophile choisi dans le groupe constitué par les polysorbates, les polyéthylèneglycols éthoxylés, les éthers étoxylés et les esters étoxylés,
- un phospholipide comme composant amphiphile,
- au moins un agent de consistance,
- au moins un hydrocarbure saturé choisi parmi la paraffine et les polysiloxanes comme composant occlusif ou, en alternative, une huile ou une cire synthétique ou naturelle,
- au moins un additif inorganique et/ou organique comprenant un sel ou un liquide ionique, et
- au moins un oligonucléotide,
**caractérisée en ce que**
- la composition pharmaceutique est une émulsion eau dans huile eau (W O W) ou eau dans huile (W-O) avec une phase aqueuse dispersée, interne, discontinue, avec addition d'ions magnésium ou sodium, et l'oligonucléotide est une enzyme ADN choisie dans le groupe constitué par les enzymes ADN hgd1 à hgd70 et td1 à td78.

2. Composition pharmaceutique selon la revendication 1 comprenant au moins un humectant et/ou un conservateur.

3. Composition pharmaceutique selon la revendication 1, **caractérisée en ce que** le sel comprend une partie cationique avec Na ou Mg, ou une combinaison de ces éléments.

4. Composition pharmaceutique selon la revendication 3, **caractérisée en ce que** le liquide ionique comprend une combinaison desdits éléments avec des cations organiques.

5. Composition pharmaceutique selon la revendication 1, **caractérisée en ce que** ledit au moins un émulsifiant hydrophile est choisi dans le groupe constitué par les stéaréths, les laureths et les cétéaréths.

6. Composition pharmaceutique selon la revendication 1, **caractérisée en ce que** le phospholipide du composant amphiphile est la lécithine.

7. Composition pharmaceutique selon la revendication 1, **caractérisée en ce que** la composition comprend au moins un humectant, ledit au moins un humectant étant choisi dans le groupe constitué par le glycérol, les polyols, les osmolytes.
